(19) Europäisches Patentamt
European Patent Office
Office européen des brevets

(11) Publication number: **0 347 864**
**A2**

(12) **EUROPEAN PATENT APPLICATION**

(21) Application number: **89111246.8**

(22) Date of filing: **20.06.89**

(51) Int. Cl.[4]: **A61K 33/34 , A61K 33/30 , A61K 31/525 , A61K 31/455 , A61K 31/375 , A61K 31/105 , A61K 31/095 , A61K 31/68 , A61K 31/505 , A61K 31/205 , A61K 31/00**

(30) Priority: **24.06.88 ZA 884556**

(43) Date of publication of application:
**27.12.89 Bulletin 89/52**

(84) Designated Contracting States:
**AT BE CH DE ES FR GB GR IT LI LU NL SE**

(71) Applicant: **Strydom, Andries Johannes Cornelus**
**21 Dersley Street Bayswater**
**Bloemfontein Orange Free State(ZA)**

(72) Inventor: **Strydom, Andries Johannes Cornelus**
**21 Dersley Street Bayswater**
**Bloemfontein Orange Free State(ZA)**

(74) Representative: **Lewinsky, Dietrich, Dipl.-Ing., Dipl.-oec. publ. et al**
**Patentanwälte Dipl.-Ing., Dipl.-oec. publ.**
**Dietriech Lewinsky Dipl.-ing Reiner Prietsch**
**Gotthardstrasse 81**
**D-8000 München 21(DE)**

(54) **Anti-atherogenic agents.**

(57) As an anti-atherogenic agent, a substance or composition capable of lowering, in human plasma, the concentration of free sulfhydryl groups forming part of homocysteine and/or cysteine molecules, thereby inhibiting directly the formation of atherosclerotic plaques in the human body.

EP 0 347 864 A2

THIS INVENTION relates to anti-atherogenic agents.

According to a first aspect of this invention, there is provided, as an anti-atherogenic agent, a substance or composition capable of lowering, in human plasma, the concentration of free sulfhydryl groups forming part of homocysteine and/or cysteine molecules, thereby inhibiting directly the formation of atherosclerotic plaques in the human body.

According to a second aspect of the invention, there is provided, as an anti-atherogenic agent, a substance or composition capable of inhibiting, ie hindering and/or lowering, the deleterious disulfide exchange reaction of homocysteine and/or cysteine with structural/functional proteins in arterial walls, thereby inhibiting directly the formation of atherosclerotic plaques in the human body.

The substance or composition may then comprise at least one compound capable of reacting directly or indirectly with the arterial wall cells and/or with the structural/functional proteins of the arterial wall cement, ie intercellular and/or cell-substrate cement, so as to prevent or hinder the homocysteine and/or cysteine molecule(s) from structurally damaging the group of structural/functional proteins such as fibronectin, in general known as glycoproteins, thereby to reduce directly or indirectly the mechanical and/or chemical stress on susceptible disulfide bonds of the structural/functional proteins.

According to a third aspect of the invention, there is provided, as an anti-atherogenic agent, the combination of (i) a substance or compositon capable of lowering, in human plasma, the concentration of free sulfhydryl groups forming part of homocysteine and/or cysteine molecules, and (ii) a substance or composition capable of inhibiting the deleterious disulfide exchange reaction of homocysteine and/or cysteine with structural/functional proteins in arterial walls, thereby inhibiting directly the formation of atherosclerotic plaques in the human body.

The Applicant has found that the amino acid homocysteine [$H_2NCH(COOH)CH_2CH_2SH$], and to a lesser extent, the amino acid cysteine [$H_2NCH(COOH)CH_2SH$], are atherogenic agents in the human body, and that their atherogenic capability resides in their free sulfhydryl [-SH] groups. These molecules exert their atherogenic capability via the mechanism known as a disulfide exchange reaction, viz.

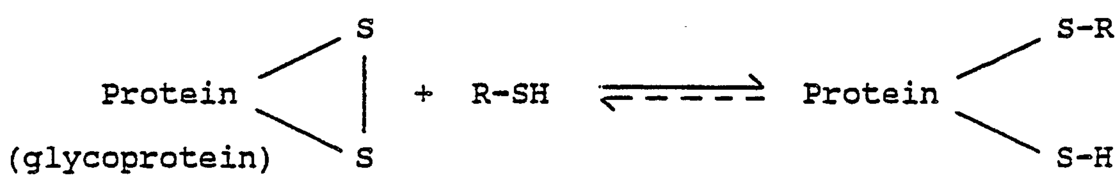

where R-SH = homocysteine and/or cysteine.

Furthermore, the Applicant has found that: (a) the reaction is critically dependent on the amount or degree of mechanical and/or chemical stress that is exerted on the relevant glycoprotein(s) and (b) that the reaction is more specific (irreversible) for homocysteine than for cysteine.

Hence, a substance or composition which lowers the concentration of the free sulfhydryl group of homocysteine and/or cysteine will act as an anti-atherogenic agent capable of inhibiting directly atherosclerotic plaque formation in the human body. This lowering of the homocysteine and/or cysteine free sulfhydryl group concentration in the plasma can be effected either by the substance or composition reacting with, or causing a reaction of, the free sulfhydryl group, thereby converting it into another group and hence lowering or hindering its activity, or by inducing reduction of free homocysteine and/or cysteine levels in plasma. These homocysteine and/or cysteine levels can be "normal" levels, ie those of healthy people, or elevated levels, ie those of homocystinuric people or animals.

The substance or composition may hence be, or include, at least one oxidizing agent and/or an oxidizing promotion/catalyzing agent, and must as such be of optimal synergistical composition, so as to include, for example, at least one cupric and/or zinc salt; at least one vitamin such as vitamin $B_2$ (riboflavin), niacin (nicotinamide), dehydroascorbic acid and/or an appropriate derivative of these vitamins; at least one compound from the group of quinones or quinone derivatives, such as ubiquinone, vitamin K, or vitamin K analogues like menadione; at least one compound from the general broad group of substances known as bioflavonoids (or flavonoids) such as rutin, hesperidin and troxerutin; and/or at least one appropriate component or derivative of the group of steroid hormones such as estradiol or dehydro-epiandrosterone (DHEA) and its sulfate ester, dehydro-epiandrosterone sulfate (DHEAS), the latter two being major secretory products of the human adrenal gland.

Instead, or additionally, the substance or composition can be, or include, at least one disulfide compound of the formula X-S-S-Y where X-SH and Y-SH are the same or different and each is a sulfhydryl containing compound, and may be administered in the form X-SH alone, Y-SH alone, or as X-SH plus Y-SH

to form X-S-S-Y within the body by oxidation, or the substance may be administered initially in the form X-S-S-Y. Each of the three forms, viz X-SH, Y-SH and X-S-S-Y is capable of reacting with homocysteine (or cysteine) in the body (or plasma) to form, for example, mixed disulfides of the formula $H_2NCH(COOH)$-$CH_2CH_2$-S-S-X and/or $H_2NCH(COOH)CH_2CH_2$-S-S-Y. Suitable sulfhydryl and/or disulfide containing compounds are believed to include cystamine, cysteamine, allyl-propyl disulfide, diallyl disulfide, glutathione (oxidized or reduced) and even cyst(e)ine, or the like.

Instead, or additionally, the substance or composition may be, or include, at lease one compound capable of decreasing homocysteine and/or cysteine levels per se in plasma, by its function as a co-factor or co-substrate in the transsulfuration pathway or methionine metabolism. Examples of such compounds are Vitamin $B_6$, Vitamin $B_{12}$, folic acid, co-substrates such as betaine and choline, or their metabolic precursors.

Still further, the substance or composition may be, or include, salts like magnesium chloride and/or at least one compound from the group of substances known as bioflavonoids (or flavonoids) like hydroxyethylrutoside and/or at least one compound from the group of substances that have a vasodilatory or anti-vasoactive action, or for this matter, arterial cell relaxation action, for example, a substance like cilazapril, an angiotensin converting enzyme (ACE) inhibitor, ie all of them substances that are believed to react directly or indirectly with relevant arterial wall cells and/or relevant structural/functional proteins of the arterial wall cell-cement, so as to effectively reduce directly or indirectly the mechanical and/or chemical stress on susceptible disulfide bonds of the relevant structural/functional proteins like fibronectin in the arterial wall cell-cement, thereby protecting them from the deleterious disulfide exchange reactions with homocysteine and/or cysteine, as described above.

The substance or composition may be administered on its own, or in admixture with a pharmaceutically acceptable adjunct, and can typically be administered orally, in which case it can be in the form of a tablet including a suitable binder or carrier, capsule, syrup including a suitable base, or the like.

It can be administered at any desired dosage rate, within the so-called 'therapeutical window' range. For example, it can be administered at relatively low dosages to healthy persons having "normal" homocysteine and/or cysteine levels, eg prophylactic or preventative dosages, or at relatively high therapeutic dosages to homocystinuric persons. For example, folic acid can be administered at a daily dosage rate of about 5 milligram ("mgm"), pyridoxine hydrochloride (Vitamin $B_6$) at a daily rate of about 50 mgm, cyanocobalamin (Vitamin $B_{12}$) at a daily rate of about 0,05 mgm, and choline or betaine at a daily rate of about 5 gm.

According to a fourth aspect of the invention, there is provided the use of an active substance or composition capable of lowering, in plasma, the concentration of free sulfhydryl groups forming part of homocysteine and/or cysteine molecules, thereby to inhibit directly atherosclerosis in the human body, for the manufacturing of a pharmaceutical to inhibit atherosclerosis.

According to a fifth aspect of the invention, there is provided the use of an active substance or composition capable of inhibiting, ie hindering or lowering, the deleterious disulfide exchange reaction of homocysteine and/or cysteine with structural/functional proteins in the arterial wall, thereby directly inhibiting atherosclerosis in the human body, for the manufacturing of a pharmaceutical to inhibit atherosclerosis.

The invention extends also to the use of the combination of (i) an active substance or composition capable of lowering, in plasma, the concentration of free sulfhydryl groups forming part of homocysteine and/or cysteine molecules, and (ii) an active substance or composition capable of inhibiting the deleterious disulfide exchange reaction of homocysteine and/or cysteine with structural/functional proteins in the arterial wall, thereby directly inhibiting atherosclerosis in the human body, for the manufacturing of a pharmaceutical to inhibit atherosclerosis.

According to a sixth aspect of the invention, there is provided the use of a substance or composition capable of lowering, in plasma, the concentration of free sulfhydryl groups forming part of homocysteine and/or cysteine molecules, to inhibit atherosclerosis in the human body.

According to a seventh aspect of the invention, there is provided the use of a substance or composition capable of inhibiting, ie hindering or lowering, the deleterious disulfide exchange reaction of homocysteine and/or cysteine with structural/functional proteins in the arterial wall, to inhibit atherosclerosis in the human body.

The invention extends also to the use of both, or the combination of, said substance or composition capable of lowering the free sulfhydryl groups, and said substance or composition capable of inhibiting the deleterious disulfide exchange reaction, to inhibit atherosclerosis in the human body.

According to a eighth aspect of the invention, there is provided a method of inhibiting atherosclerosis in a human body, which comprises administering to the body an effective amount of a substance or composition capable of lowering the concentration of free sulfhydryl groups forming part of homocysteine and/or cysteine molecules in the body plasma, thereby to inhibit directly atherosclerosis in the body.

According to a ninth aspect of the invention, there is provided a method of inhibiting atherosclerosis in

an human body which comprises administering to the body an effective amount of a substance or composition capable of inhibiting the deleterious disulfide exchange reaction of homocysteine and/or cysteine with structural/functional proteins in the arterial wall, thereby inhibiting directly atherosclerosis in the body.

The invention extends also to the use of both, ie the combination of, said substance or composition capable of lowering the concentration of free sulfhydryl groups, and said substance capable of inhibiting the deleterious disulfide exchange reaction, in the method of atherosclerosis inhibition.

The method may also comprise monitoring plasma total homocysteine, homocysteine-cysteine mixed disulfide and/or cyst(e)ine levels, for example, by means of an amino acid analyser, using among others a procedure based on that set out under the heading 'ANALYTICAL PROCEDURE' hereunder. The method may further comprise administering sufficient of the substance or composition to control the body plasma homocysteine or cysteine level at a desired value. For example, by taking the normal fasting circulation plasma homocysteine-cysteine mixed disulfide level (X-Dis) as a marker, the free plasma homocysteine level must be controlled to such an extent, that the fasting concentration of this marker (X-Dis) is reduced to reach ideally a value <1μmol/L plasma [the normal concentration of this marker (X-Dis) is usually between 1 and 7μmol/L].

The method further comprises the administering of sufficient of the substance or composition to control the deleterious disulfide exchange reaction of homocysteine and/or cysteine with structural/functional proteins in the arterial wall. This deleterious disulfide exchange reaction can be evaluated and/or monitored by the following non-limiting procedure or example:
A long-term moderate homocystinemia (slightly elevated plasma homocysteine levels) can be induced in appropriate experimental animals by parental administration of methionine. The particular substance or composition may then be administered at a selected or comparative dosage rate for an appropriate period of time in these animals with elevated plasma homocysteine levels. The animals may then be killed at the end of the experiment and an appropriate segment of the aorta removed and investigated for a beneficial difference in endothelial permeability by light and/or scanning electron microscopy as compared to an appropriate control segment from animals which have received no such protective substance or composition. In this fashion, an appropriate dosage rate for dosing the human body with that substance can be determined.

Finally, the method may also comprise the optimizing of the inhibition of atherosclerosis in the human body through the use of a synergistical substance or composition for an appropriate period of time. For example, a substance or composition, like some of the vitamins listed hereinbefore, may be administered to the human body during its whole lifespan, ie from infant life until old age, so as to maintain the body plasma homocysteine at a desired value, and may additionally be fortified with a substance or composition that is capable of hindering or lowering the deleterious disulfide exchange reaction of homocysteine and/or cysteine with structural/functional proteins in the arterial wall, as and/or when needed.

The invention will now be described by way of example with reference to the following non-limiting test.

Vitamin $B_6$ (pyridoxal phosphate) levels of about 330 subjects were determined by means of a high pressure liquid chromatographic method. The subjects were grouped into categories according to their plasma $B_6$ reading, viz <5 ng $B_6$/ml, 5-7 ng/ml, 7-10 ng/ml, 10-15 ng/ml, 15-20 ng/ml, and >20 ng/ml. The <5 ng/ml group contained subjects known to have low or subclinical $B_6$ levels, while the >20 ng/ml group were identified as having been supplemented with $B_6$, eg in medication. For each group, the total plasma homocysteine levels of a randomized subgroup (10 or more subjects) were determined with an amino acid analizer (using a procedure based on that set out under the heading "ANALYTICAL PROCEDURE" hereunder) and the mean or average calculated for each group. These values were plotted against the groups as indicated in the attached graph (see page 13).

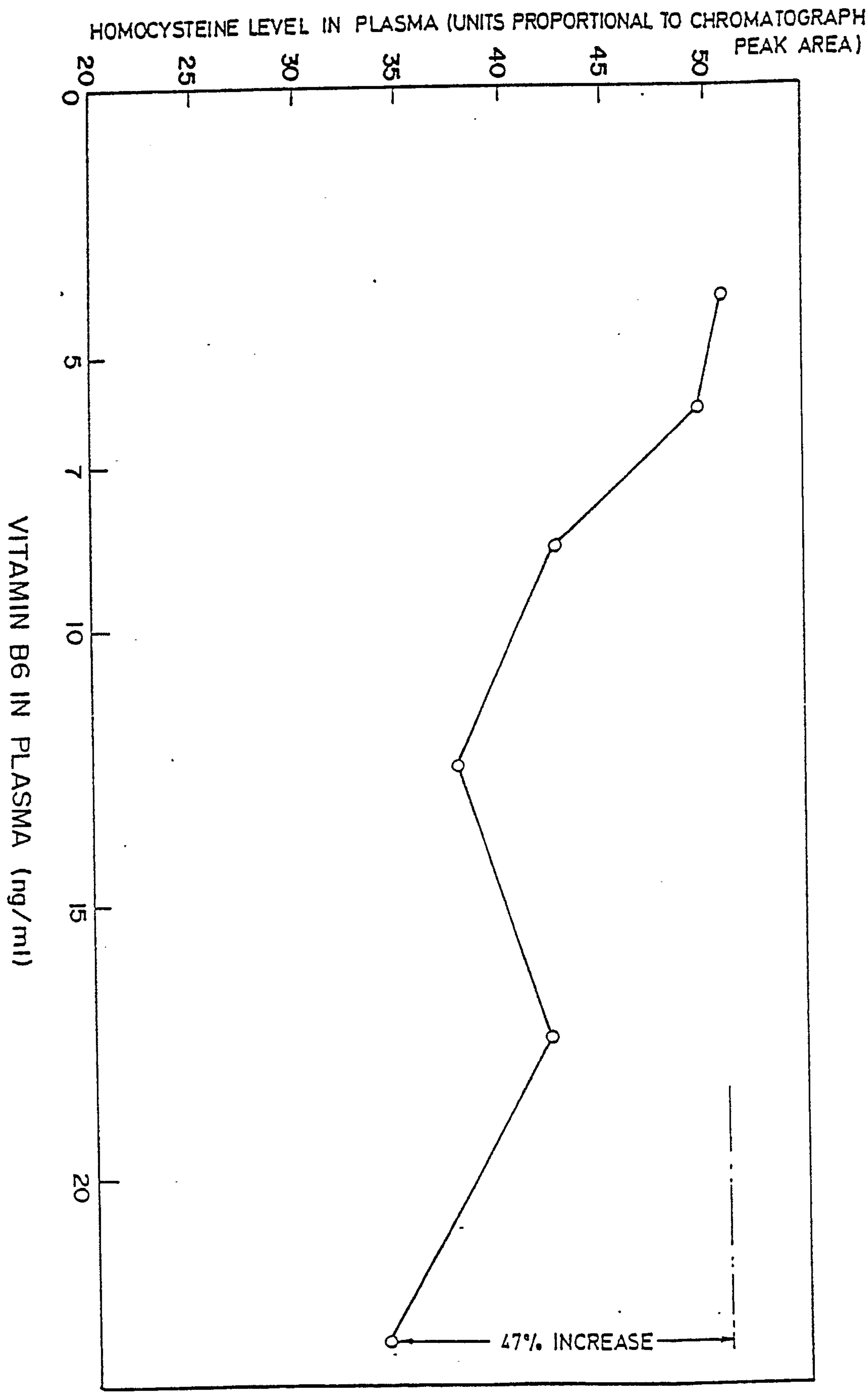
HOMOCYSTEINE LEVEL IN PLASMA (UNITS PROPORTIONAL TO CHROMATOGRAPH PEAK AREA)
20
25
30
35
40
45
50
0
5
7
10
15
20
VITAMIN B6 IN PLASMA (ng/ml)
47% INCREASE

From this graph it can clearly be seen that an approximately 4-fold increase in $B_6$ level leads to almost a 50% reduction in homocysteine levels, hence confirming that by administering a substance in accordance with the invention, such as Vitamin $B_6$, homocysteine levels are lowered. When these lowered levels are maintained for a long period of time (for example life-long), this may lead to a concomitant degree of inhibition of atherosclerosis, ie reduction in ischaemic diseases, as a result of the following mechanism identified by the Applicant: at low or "normal" homocysteine levels in the plasma, there is normally little or only a low rate of atherosclerotic plaque formation of endothelial permeability damage to the arterial wall. However, at elevated homocysteine levels, as would be evidenced in homocystinuric patients, atherosclerosis takes place at an accelerated rate as a result of disulphide exchange of susceptible bonds in cell-to-cell or cell-to-substrate adhesive proteins, and/or to cell membrane proteins and/or to cell connective tissue proteins due to the -SH group of homocysteine, resulting in, amongst others, loss of the structural/functional properties of the proteins concerned, for example, fibronectin and/or proteoglycans, with an concomitant change or damage to the endothelial permeability, or for that matter, the endothelial cell integrity of the arterial wall and/or other arterial wall cells and which may eventually lead to, amongst other pathological changes, endothelial denudution or desquamation of the arterial wall. Furthermore, even in persons having "normal" homocysteine levels, when the arterial cells are under additional mechanical and/or chemical stress, eg as normally happens in arteries in proximity to the heart, such as the coronary arteries, due to, amongst others, rheological factors, and/or in smokers, and/or in persons with high blood pressure, high total serum cholesterol levels and/or other forms of stress such as are induced by inflammation and/or vasoactive substances, or such as do occur in type A personalities, "normal" homocysteine levels also initiate atherosclerosis by the mechanism set out hereinbefore, with the resultant atherosclerosis also being enhanced by these other factors, or in relevant arteries.

The same applies, in essence, to cysteine, although according to a concentration criterion, only less efficiently.

An important aspect of the identified mechanism is (a) that the coronary artery is normally already continuously exposed to so-called "extra mechanical stress" due to pulsatile flood flow from the heart. According to the above-identified mechanism, this part of the artery (or adjacent parts) is more prone to occlusive atherosclerotic plaque development than the peripheral or cerebral arteries at normal plasma homocysteine levels; and (b) that according to the identified mechanism, homocysteine at 'normal' as well as elevated plasma homocysteine levels, leads to the most important initiation event in the aetiology of atherosclerosis, viz increased or damaged endothelial permeability. The difference between 'normal' and elevated plasma homocysteine levels, is not only related to a difference in the rate of endothelial permeability damage, but also to the character of ensuing atherosclerotic plaques, viz mainly ischaemic heart disease at 'normal' levels and essentially occlusive peripheral and cerebral arterial diseases, as well as thrombophlebitis at moderate to highly elevated plasma homocysteine levels. Additionally, when the abovementioned mechanical and/or chemical stress factors are increased at 'normal' plasma homocysteine levels, the ensuing occlusive complications may be both in the heart, periferal and cerebral arteries.

Hence, by controlling homocysteine free sulfhydryl group levels, and/or the deleterious disulfide exchange reaction of homocysteine and/or cysteine the Applicant believes that atherosclerosis can be controlled or inhibited.

ANALYTICAL PROCEDURE

Method: 1) The plasma was prepared according to a standard procedure (ie blood cooled immediately after venepuncture and plasma separated within 1 hour). The plasma was then conveniently stored frozen for any length of time, if necessary.

2) Homocysteine was released from the plasma by dithiotreitol treatment and deproteinized with sulfosalicylic acid. After an adjustment of the pH, the sample was analyzed directly. According to the literature however, it is actually this step that must conventionally be avoided (due to instability) and homocysteine must first be derivatized before analysis (to the S-carboxymethyl derivative for example). Apart from being laborious, the latter step however also leads to losses in yield.

Direct analysis were however performed under the following precautionary conditions: a) Due to low concentrations and poor colour yield with ninhydrin, homocysteine must be separated quite well from all contaminating substances. For this purpose the analyzer must have excellent resolving capabilities, high sensitivity, as well as an excellent on-line chromatography data system. b) Losses do occur however on the column, especially in the plasma range from 1 to 5 umol/L. This was however compensated for by the use

of a carefully constructed standard curve. A special short program for the separation of homocysteine was developed, with plasma homocysteine yields that are in good agreement with the capillary gas chromatography -mass spectroscopy technique developed recently.

## Claims

1. As an anti-atherogenic agent, a substance or composition capable of lowering, in human plasma, the concentration of free sulfhydryl groups forming part of homocysteine and/or cysteine molecules, thereby inhibiting directly the formation of atherosclerotic plaques in the human body.

2. An anti-atherogenic agent according to Claim 1, characterized in that the substance or composition comprises at least one oxidizing agent and/or an oxidizing promotion/catalyzing agent.

3. An anti-atherogenic agent according to Claim 2, characterized in that the substance or compound is selected from a cupric salt, a zinc salt, vitamin $B_2$, niacin, dihydroascorbic acid, quinone, a quinone derivative, a bioflavonoid compound, hesperidin, a steroid hormone, and a steroid hormone derivative.

4. An anti-atherogenic agent according to Claim 1, characterized in that the substance or composition comprises at least one disulfide compound of the formula X-S-S-Y where X-SH and Y-SH are the same or different and are each a sulfhydryl containing compound, with X-S-S-Y being capable of reacting with homocysteine and/or cysteine in the human body to form mixed disulfides.

5. An anti-atherogenic agent according to Claim 1, characterized in that the substance or composition comprises a compound X-SH and/or Y-SH, where X and Y are the same or different, and are capable of forming the disulfide compound X-S-S-Y within the body, with X-SH, Y-SH and X-S-S-Y being capable of reacting with homocysteine and/or cysteine in the human body to form mixed disulfides.

6. An anti-atherogenic agent according to Claim 1, characterized in that the substance or composition comprises at lease one compound capable of decreasing homocysteine and/or cysteine levels per se in plasma, by its function as a co-factor or co-substrate in the transsulfuration pathway of methionine metabolism.

7. An anti-atherogenic agent according to Claim 6, characterized in that the compound is selected from Vitamin $B_6$, Vitamin $B_{12}$, folic acid, betaine, choline, or their metabolic precursors.

8. As an anti-atherogenic agent, a substance or composition capable of inhibiting the deleterious disulfide exchange reaction of homocysteine and/or cysteine with structural/functional proteins in arterial walls, thereby inhibiting directly the formation of atherosclerotic plaques in the himan body.

9. An anti-atherogenic agent according to Claim 8, characterized in that the substance or composition comprises at lease one compound capable of reacting directly or indirectly with the arterial wall cells and/or with the structural/functional proteins of the arterial wall cell-cement, thereby to reduce directly or indirectly the mechanical and/or chemical stress on susceptible disulfide bonds of the structural/functional proteins.

10. An anti-atherogenic agent according to Claim 9, characterized in that the compound is selected from magnesium chloride, a bioflavonoid, a vasodilator, and an arterial cell relaxation agent.

11. As an anti-atherogenic agent, the combination of (i) a substance or composition capable of lowering, in human plasma, the concentration of free sulfhydryl groups forming part of homocysteine and/or cysteine molecules, and (ii) a substance or composition capable of inhibiting the deleterious disulfide exchange reaction of homocysteine and/or cysteine with structural/functional proteins in arterial walls, thereby to inhibit directly the formation of atherosclerotic plaques in the human body.

12. The use of a substance or composition capable of lowering, in body plasma, the concentration of free sulfhydryl groups forming part of homocysteine and/or cysteine molecules, thereby to inhibit directly atherosclerosis in the human body, for the manufacture of a pharmaceutical to inhibit atherosclerosis.

13. The use of a substance or composition capable of inhibiting the deleterious disulfide exchange reaction of homocysteine and/or cysteine with structural/functional proteins in the arterial wall, thereby inhibiting directly atherosclerosis in the human body, for the manufacture of a pharmaceutical to inhibit atherosclerosis.

14. The use of a substance or composition capable of lowering, in body plasma, the concentration of free sulfhydryl groups forming part of homocysteine and/or cysteine molecules, to inhibit atherosclerosis in the human body.

15. The use of a substance or composition capable of inhibiting the deleterious disulfide exchange reaction or homocysteine and/or cysteine with structural/functional proteins in arterial walls, to inhibit atherosclerosis in the human body.